(19) Europäisches Patentamt
European Patent Office
Office européen des brevets

(11) Publication number: 0 525 573 A1

(12) **EUROPEAN PATENT APPLICATION**

(21) Application number: **92112362.6**

(22) Date of filing: **20.07.92**

(51) Int. Cl.[5]: **C07F 9/38**, A61K 31/695, C08G 77/30, C08G 79/00, C07F 9/40, A61K 31/66, A61K 31/80

(30) Priority: **29.07.91 JP 210483/91**

(43) Date of publication of application: **03.02.93 Bulletin 93/05**

(84) Designated Contracting States: **AT BE CH DE DK ES FR GB GR IT LI LU MC NL PT SE**

(71) Applicant: **SANWA KAGAKU KENKYUSHO CO., LTD.**
**No. 35, Higashi-sotobori-cho**
**Higashi-ku Nagoya-shi Aichi-ken(JP)**

(72) Inventor: **Kurono, Masayasu, Sanwa Kagaku Kenkyusho Co.Ltd.**
**35, Higashi-sotobori-cho**
**Higashi-ku, Nagoya, Aichi-ken(JP)**
Inventor: **Kondo, Yasuaki, Sanwa Kagaku Kenkyusho Co.Ltd.**
**35, Higashi-sotobori-cho**
**Higashi-ku, Nagoya, Aichi-ken(JP)**
Inventor: **Baba, Yutaka, Sanwa Kagaku Kenkyusho Co.Ltd.**
**35, Higashi-sotobori-cho**
**Higashi-ku, Nagoya, Aichi-ken(JP)**
Inventor: **Iwata, Noriyuki, Sanwa Kagaku Kenkyusho Co.Ltd.**
**35, Higashi-sotobori-cho**
**Higashi-ku, Nagoya, Aichi-ken(JP)**
Inventor: **Mitani, Takahiko, Sanwa Kagaku Kenkyusho Co.Ltd.**
**35, Higashi-sotobori-cho**
**Higashi-ku, Nagoya, Aichi-ken(JP)**
Inventor: **Ishiwata, Yoshiro, Sanwa Kagaku Kenkyusho Co.Ltd.**
**35, Higashi-sotobori-cho**
**Higashi-ku, Nagoya, Aichi-ken(JP)**
Inventor: **Sawai, Kiichi, Sanwa Kagaku Kenkyusho Co.Ltd.**
**35, Higashi-sotobori-cho**
**Higashi-ku, Nagoya, Aichi-ken(JP)**

(74) Representative: **Wächtershäuser, Günter, Dr.**
**Tal 29**
**W-8000 München 2(DE)**

(54) **Organogermanium and organosilicon compounds, process for the preparation of the same and use thereof.**

(57) There is disclosed organogermanium and organosilicon compounds shown by the formula of

$$[0_{1/2})_3Y\text{-}C(R_1)(R_2)\text{-}C(R_3)(R_4)\text{-}(CH_2)_m\text{-}P(=0)(0R_5)_2]_n$$

wherein $R_1$, $R_2$, $R_3$, $R_4$ and $R_5$ are same or different and each represents hydrogen atom, an lower alkyl group with possible substituent(s) or phenyl radical with possible substituent(s), respectively; Y is germanium or silicon atom; $_m$ is an integer of 0 or more; and $_n$ is an integer of 2 or more,
and salts thereof, process for the preparation of the same as well as its use. The compound is administered to adjust immunoresponse.

EP 0 525 573 A1

The present invention relates to organogermanium and organosilicon compounds (polymers), process for the preparation of same and use thereof, as a pharmacological composition and more particularly as an immune modulating agent.

Organogermanium compounds have been watched with great interest in recent years, since those show various biological activities.

For instance, a compound represented by the formula of

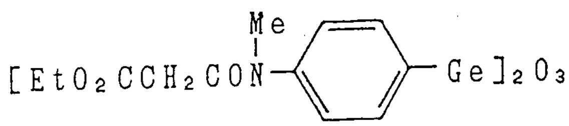

wherein Et is ethyl radical and Me is methyl radical, and known in the art as "Y-9577" shows an antiinflammatory activity [Jap. Pat. Nos. Sho 56 (A.D. 1981) - 45492(A), 99418(A), 99419(A) and 108708(A)].

Another compound represented by the formula of

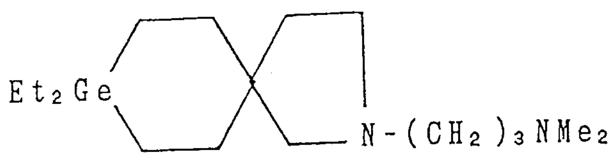

wherein Et and Me have the meaning as referred to, and known in the art as "Spirogermanium" shows anti-tumor action ["Cancer Res.", Vol. 42, page 2852 (1982)].

While, the present inventors have also studied and investigated on organogermanium compounds and reported that 3-oxygermylpropionic acid derivatives show an excellent immune modulating activity [Jap. Pat. No. Sho 61 (A.D. 1986) - 151173(A) which corresponds to USP 4,889,715 and EP-0 186 505(A2)], and that 2-oxygermylethanesulfonic acid derivatives show also an excellent immune modulating activity and anti-tumor activity [Jap. Pat. No. Hei 3 (A.D. 1991) - 24094 which corresponds to USP 5,008,416 and EP-0 404 062(A2)].

Organosilicon compounds have also watched with great interest, since those show various biological activities similar to the organogermanium compounds.

For instance, a compound represented by the formula

$Me_3SiCH_2CH_2SCH_2CH_2NH_2$

wherein Me has the meanings as referred to, shows an anti-tumor activity [Jap. Pat. No. Sho 59 (A.D. 1984) - 70692(A)]

In spite of that various biological activities have been reported as referred to on various organogermanium and organosilicon compounds, it has not been sufficiently elucidated as to a co-relation between a structure and biological activities of the compounds as well as mechanisms, based on which the compounds generate a specific biological activity.

The present inventors have so assumed through the studies and investigations on the various prior arts as well as their experiments that a remarkable difference shall appear on a kind of biological activity and its intensity, due to a difference in structure. This means that if a novel organogermanium or organosilicon compound is prepared to investigate its biological activities, a discovery of novel biological activity(ies) or known but increased biological activity can be expected, in addition to a contribution in elucidation of the co-relation between the structure and biological activities of the compounds as well as mechanisms, based on which the compounds generate those specific biological activity(ies).

A fundamental object of the invention is, therefore, to provide novel organogermanium and organosilicon compounds showing various and excellent biological activities.

An additional object of the invention is to provide a process for the preparation of such organogermanium and organosilicon compounds.

The inventors have paid their possible efforts to develop novel organogermanium and organosilicon compounds showing an excellent biological activity(ies) to finally find out that organogermanium and organosilicon compounds with phosphonic acid residue in its molecule are preferable, so that the invention

was established.

The organogermanium and organosilicon compounds according to the invention are shown by the formula of

$[0_{1/2})_3Y\text{-}C(R_1)(R_2)\text{-}C(R_3)(R_4)\text{-}(CH_2)_m\text{-}P(=0)(0R_5)_2]_n$

wherein $R_1$, $R_2$, $R_3$, $R_4$ and $R_5$ are same or different and each represents hydrogen atom, an lower alkyl group with possible substituent(s) or phenyl radical with possible substituent(s), respectively; Y is germanium or silicon atom; $_m$ is an integer of 0 or more; and $_n$ is an integer of 2 or more.

A salt of the compounds is also useful.

Followings shall be listed as the lower alkyl group with possible substituent(s): straight-chain and branched-chain saturated hydrocarbon groups with 1 - 6 carbon atoms, such as methyl, ethyl, propyl, isopropyl, butyl, isobutyl, s-butyl, t-butyl, pentyl, hexyl radicals and the like; halogenoalkyl group such as fluoromethyl, chloromethyl, difluoromethyl, trifluoromethyl, fluoroethyl, chloroethyl, bromoethyl, difluoroethyl, dichloroethyl, trifluoroethyl, trichloroethyl, fluoropropyl, chloropropyl, difluoropropyl, trifluoropropyl radicals and the like; benzyl group such as benzyl, o-chlorobenzyl, m-chlorobenzyl, p-chlorobenzyl, p-nitrobenzyl, p-methoxybenzyl radicals and the like. Following shall be listed as the phenyl group with possible substituent-(s) : phenyl, o-chlorophenyl, m-chlorophenyl, p-chlorophenyl, o-fluorophenyl, m-fluorophenyl, p-fluorophenyl, o-bromophenyl, m-bromophenyl p-bromophenyl, 2,3-dichlorophenyl, 2,4-dichlorophenyl, 2,5-dichlorophenyl, 2,6-dichlorophenyl, 3,4-dichlorophenyl, 3,5-dichlorophenyl, 2,3-difluorophenyl, 2,4-difluorophenyl, 2,5-difluorophenyl, 2,6-difluorophenyl, 3,4-difluorophenyl, 3,5-difluorophenyl, o-hydroxyphenyl, m-hydroxyphenyl, p-hydroxyphenyl, 2,3-dihydroxyphenyl, 2,4-dihydroxyphenyl, 2,5-dihydroxyphenyl, 2,6-dihydroxyphenyl, 3,4-dihydroxyphenyl, 3,5-dihydroxyphenyl, o-methoxyphenyl, m-methoxyphenyl, p-methoxyphenyl, 2,3-dimethoxyphenyl, 2,4-dimethoxyphenyl, 2,5-dimethoxyphenyl, 2,6-dimethoxyphenyl, 3,4-dimethoxyphenyl, 3,5-dimethoxyphenyl radicals and the like.

Followings can be listed as the salt of the organogermanium or organosilicon compound, which is acceptable from pharmacological view points : salts with a metal such as sodium, potassium, calcium, magnesium or the like; salts with an organic base such as ammonia, methylamine, dimethylamine, ethanolamine, meglumine, dicyclohexylamine, benzylamine or the like; salts with a basic amino acid such as lysine, arginine, ornithine, histidine, tryptophan, oxylysine, oxyarginine, homoarginine and the like.

According to the invention, the organogermanium, organosilicon compound or salt thereof can be prepared by reacting a trihalogermane or trihalosilane shown by the formula of

$X_3YH$

wherein X and Y have the meanings as referred to, with a phosphonic acid derivative shown by the formula of

$(R_1)(R_2)C=C(R_3)\text{-}(CH_2)_m\text{-}P(=0)(0R_4)_2$

wherein $R_1$, $R_2$, $R_3$ and $R_4$ have the meanings as referred to,
subjecting to hydrolysis the resulting trihalogermylphosphonic acid derivative or trihalosilylphosphonic acid derivative shown by the formula of

$X_3Y\text{-}C(R_1)(R_2)\text{-}C(R_3)H\text{-}(CH_2)_m\text{-}P(=0)(0R_4)_2$

wherein X, Y, $R_1$, $R_2$, $R_3$ and $R_4$ have the meanings as referred to,
and if necessary, converting the derivative into its salt.

In the above process, the reaction between the trihalogermane or trihalosilane and phosphonic acid derivative can be carried out in the presence or absence of a solvent and in the presence or absence of a catalyst to stir the reactants at a temperature of 0 - 200 °C, and preferably in a sealed tube. The resulting trihalogermylalkanephosphonic acid derivative or trihalosilylalkanephosphonic acid derivative can easily be isolated in a conventional manner but the isolation is not always required, so that the next step of hydrolysis can be carried out directly.

Further, according to the invention, a compound shown by the formula of

$[0_{1/2})_3Ge\text{-}C(R_6)(R_7)\text{-}C(R_8)(R_9)\text{-}(CH_2)_m\text{-}P(=0)(0R_{10})_2]_n$

wherein $R_6$, $R_7$, $R_8$, $R_9$ and $R_{10}$ are same or different and each represents hydrogen atom, an lower alkyl group with possible substituent(s), or phenyl radical with possible substituent(s), respectively; Y is germanium or silicon atom; $_m$ is an integer of 0 or more; and $_n$ is an integer of 2 or more, or a salt thereof can be prepared by reacting dihalogermane shown by the formula of

$X_2Ge$

wherein X is halogen atom, with a haloalkanephosphonic acid derivative shown by the formula of

$X\text{-}C(R_6)(R_7)\text{-}C(R_8)(R_9)\text{-}(CH_2)_m\text{-}P(=0)(0R_{10})_2$

wherein $R_6$, $R_7$, $R_8$, $R_9$, $R_{10}$ and $_m$ have the meanings as referred to, subjecting the resulting trihalogermylalkanephosphonic acid derivative shown by the formula of

$X_3Ge\text{-}C(R_6)(R_7)\text{-}C(R_8)(R_9)\text{-}(CH_2)_m\text{-}P(=0)(0R_{10})_2$

wherein $R_6$, $R_7$, $R_8$, $R_9$, $R_{10}$ and $_m$ have the meanings as referred to, and if necessary converting the derivative into its salt.

Also in the above process, the reaction between the dihalogermane and phosphonic acid derivative can be carried out in the presence or absence of a solvent and in the presence or absence of a catalyst to stir the reactants at a temperature of 0 - 200 ° C , and preferably in a sealed tube. The resulting trihalogermylalkanephosphonic acid derivative can easily be isolated in a conventional manner but the isolation is not always required, so that the next step of hydrolysis can be carried out directly.

The final step of the both processes according to the invention, namely the step for hydrolyzing the trihalogermylalkanephosphonic acid derivative or trihalosilylalkanephosphonic acid derivative can be carried out with use of water, basic aqueous solution, acidic aqueous solution or a mixture thereof with an organic solvent. An isolation of the desired oxygermylalkanephosphonic acid derivative or oxysilylalkanephosphonic acid derivative can also be carried out in a conventional manner.

The step for converting the derivative into its salt can also be carried out in a conventional manner. Namely, lyophilization or treatment using a suitable solvent such as acetone, methanol, ethanol, dioxane or the like affords the desired salt with a form of crystals or crystalline powder. Further, if the hydrolysis is carried out in an aqueous solution containing a desired base, the salt can directly be obtained.

The compound according to the invention can be made into a medicine in various form for oral or non-oral administration, by composing the same with a pharmaceutical diluent or filler. There is no specific restriction in the form of medicine and thus may be made into a tablet, capsule, granule, injection and others.

An amount of dose as the immune modulating agent depends on various factors such as symptom, age of patient and others. but the amount of 50 - 400mg/day based on the amount of the effective compound is preferable.

The invention will now be further explained in more detail and with reference to Reference Examples for preparing intermediates, Examples for preparing the final compounds, Pharmacological Test Examples as well as Medicine Preparation Examples.

Please note that in the Examples for preparing the compounds, reaction was carried out under argon, nitrogen or the like inert gas atmosphere, unless another condition shall be specifically referred to.

Reference Example 1

Diethyl 2-trichlorogermylethanephosphonate

A mixture of germanium hydroxide (428mg) and 1.8N-HCl/diethyl ether (8.00ml) was stirred for 1 hour in a sealed tube. To the resulting clear solution, was added diethyl vinylphosphonate (656mg) and the mixture was stirred for 3 days at 40 - 50 ° C in a sealed tube. The reaction mixture was concentrated and the residue was distilled under reduced pressure to give 1,00g (Yield, 72.9%) of the desired compound as a colorless oil.

| | |
|---|---|
| Boiling point : | 150 ° C (2mmHg). |
| MS spectrum (CI/DI, i-Bu) m/z : | 348, 346, 344, 342, 340 ($M^+$) 313, 311, 309, 307, 305 ($M^+$-Cl). |
| IR spectrum (ν , KBr, max.) $cm^{-1}$ : | 1220 (P=O), 1030 (P-O-C). |

$^1$H-NMR spectrum ($CDCl_3$) δ ppm : 1.36 (6H, t, J = 7.3Hz, $0CH_2CH_3$ x 2),
1.86 - 2.34 (4H, m, $GeCH_2CH_2P$),
4.15 (4H, q, J = 7.3Hz, $0CH_2CH_3$ x 2).

Reference Example 2

2-Trichlorogermylethanephosphonic acid

A mixture of diethyl 2-trichlorogermylethanephosphonate (Reference Example 1, 1.50g) and conc.-HCl (20.0ml) was refluxed for 18 hours. The reaction mixture was washed with dichloromethane (3.00ml) in 3 times and an aqueous solution was separated and evaporated to dryness under reduced pressure to give 1.16g (Yield, 92.0%) of the desired compound as colorless prisms.

IR spectrum (ν , KBr, max.) $cm^{-1}$ : 2800 - 2400 (P-OH), 1184 (P = O), 1087 (P-0-C).
$^1$H-NMR spectrum ($CDCl_3$) δ ppm : 1.24 - 1.38 (2H, m, $GeCH_2CH_2P$),
1.50 - 1.66 (2H, m, $GeVCH_2CH_2P$).

Example 1

2-Oxygermylethanephosphonic acid polymer

To a stirred solution of 2-trichlorogermylethanephosphonic acid (Reference Example 2, 1.16g) in methanol (20.0ml), was added dropwise 6.4N-ammonia/methanol (6.28ml) at 0 - 5 ° C . The precipitate was filtered and washed with methanol (5.00ml) and with ether (20.0ml), and then dried. The resulting product was purified with column chromatography (Diaion PK-216, using ion-exchanged water as eluent) to collect acidic fractions. The fractions were concentrated under reduced pressure to give 806mg (Yield, 97.7%) of the desired polymer as colorless crystals.

Melting point : > 300 ° C .

| Elementary Analysis $(C_2H_6O_{4.5}P)_n$ : | | |
|---|---|---|
| Cal. ; | C, 11.68, | H, 2.94, |
| Found ; | C, 11.77, | H, 2.65. |

IR spectrum (ν , KBr, max.) $cm^{-1}$ : 2800 - 2400 (P-OH), 1190 (P = O), 1025 (P-0-C), 810 (Ge-O).
$^1$H-NMR spectrum ($D_2O$) δ ppm : 1.24 - 1.38 (2H, m, $GeCH_2CH_2P$),
1.50 - 1.66 (2H, m, $GeCH_2CH_2P$).

Example 2

2-Oxygermylpropanephosphonic acid polymer

A mixture of germanium diiodide (1.63g) and diethyl 3-bromopropylphosphonate (1.30g) was heated for 10 min. at 150 ° C in a sealed tube. After cooling, the reaction mixture was washed with chloroform (50.0ml) and the insoluble fraction in chloroform was washed with methanol (2.0ml). To the methanol solution, was added dropwise 6.0N-ammonia/methanol solution (1.00ml) and the precipitate was filtered. The resulting product was purified with column chromatography (Diaion PK-216, using ion-exchanged water as eluent) to collect acidic fractions. The fractions were concentrated under reduced pressure to give 324mg (Yield, 29.5%) of the desired polymer as colorless crystals.

Melting point : > 300 ° C .

| Elementary Analysis $(C_3H_8O_{4.5}P \cdot 3/4\ H_2O)_n$ : | | |
|---|---|---|
| Cal. ; | C, 15.45, | H, 4.11, |
| Found ; | C, 15.23, | H, 3.67. |

IR spectrum (ν , KBr, max.) $cm^{-1}$ : - 2400 (P-OH), 1200 (P = O), 830 (Ge-O).
$^1$H-NMR spectrum ($D_2O$) δ ppm : 1.22 (2H, t, $GeCH_2CH_2CH_2P$),

1.38 - 1.58 (2H, m, $GeCH_2CH_2CH_2P$),
1.64 - 1.84 (2H, m, $GeCH_2\underline{CH_2}CH_2P$).

Example 3

2-oxysilylethanephosphonic acid polymer

A mixture of diethyl 2-triethoxysilylethanephosphonate (3.28g) and 6N-HCl (24.0ml) was refluxed for 6 hours. The reaction mixture was evaporated to dryness under reduced pressure to give 1.61g (quantitative yield) of the desired polymer as glassy crystals.

Melting point : > 300 °C .

| Elementary Analysis $(C_2H_60_{4.5}PSi)_n$ : | | |
|---|---|---|
| Calm ; | C, 14.91, | H, 3.75, |
| Found ; | C, 14.62, | H, 4.14. |

IR spectrum (ν , KBr, mad.) $cm^{-1}$ : - 2500 (P-OH), 1300 - 800 (P = O, SiO etc.).
$^1$H-NMR spectrum ($D_2O$) δ ppm : 0.80 - 2.30 (4H, m, $SiCH_2CH_2P$).

Example 4

Diethyl 2-oxysilylethanephosphonate polymer

To a stirred solution of diethyl 2-oxysilylethanephosphonate (821mg) in acetone (3.15ml), was added dropwise ion-exchanged water (3.15ml) at 0 - 5 °C . The reaction mixture was evaporated under reduced pressure at temperature lower than 40 °C to give 588mg (quantitative yield) of the desired polymer as colorless viscous oil.

IR spectrum (ν , KBr, max.) $cm^{-1}$ : 1220 (P = O), 1100 - 1000 (P-O-C, SiO etc.).
$^1$H-NMR spectrum ($D_2O$) δ ppm : 0.80 - 1.50 (2H, m $SiCH_2CH_2P$),
1.38 (6H, t, J = 7.3Hz, $0CH_2CH_3$ x 2),
1.8 - 2.2 (2H, m, $SiCH_2CH_2P$),
4.75 (4H, q, J = 7.3Hz, $O\underline{CH_2}CH_3$ x 2).

Pharmacological Test Example 1

(Effect on delayed type hypersensitivity)

a) Object

Enhancing effect of cellular immunity by the compound according to the invention was evaluated by using the delayed type hypersensitivity of tumor bearing mice as immunosuppressed mice.

b) Operation

Sarcoma-180 ascites tumor cells ($10^6$ cells) were intraperitoneally implanted to male ICR mice. After 3 hours, these mice were sensitized by intravenous injection of 2 x $10^6$ cells of sheep red blood cells (SRBC). After 4 days, 2 x $10^8$ cells of SRBC were injected to right hind foot pad, and 24 hours later, foot pad swelling was measured by a stereomicroscope.

The polymer obtained by Example 1 was dissolved in 4% bovine serum albumin solution and orally administered to a test group at the dose of 0.1, 1.0. 10.0mg/kg before 4 days from the SRBC sensitization.

c) Results and consideration

Results were shown in following Table 1. As seen therefrom, the polymer apparently restored the suppressed DTH response of tumor bearing mice. This results indicates that the polymer has immunostimulating activity.

Table 1

| Group | Dose (mg/kg) | Foot pad swelling ( #) |
|---|---|---|
| Normal | - | 117.8 ± 1.1 (***) |
| Control | - | 48.9 ± 3.2 |
| Polymer<br>ditto<br>ditto | 0.1<br>1.0<br>10.0 | 66.0 ± 8.2<br>85.0 ± 5.6 (***)<br>74.2 ± 4.8 (***) |

In the Table,
# : mean ± S.E. (x 0.01mm, n = 10), and
*** : Significant difference from control group ($p < 0.001$).

Pharmacological Test Example 2

(Effect on delayed type hypersensitivity)

a) Object

An influence of the polymer according to the invention on DTH response of normal mice was evaluated.

b) Operation

Male ICR mice were sensitized by intravenous injection of $10^6$ cells of SRBC. After 4 days, the polymer obtained by Example 1 was dissolved in 4% bovine serum albumin solution and orally administered at the dose of 0.1, 1.0. 10.0mg/kg and immediately after the administration, 2 x $10^8$ cells of SRBC were injected to right hind foot pad. After 24 hours, foot pad swelling was measured by a stereomicroscope.

c) Results and consideration

Results were shown in following Table 2. As seen therefrom, the polymer apparently restored the suppressed DTH response of normal mice. This results indicates that the polymer has immune modulating activity, the results shown in Pharmacological Test Example 1 are taken into consideration.

Table 2

| Group | Dose (mg/kg) | Foot pad swelling ( #) |
|---|---|---|
| Normal | - | 122.2 ± 11.0 |
| Polymer<br>ditto<br>ditto | 0.1<br>1.0<br>10.0 | 88.9 ± 10.2 (*)<br>67.2 ± 5.0 (***)<br>72.9 ± 9.5 (**) |

In the Table,
# : mean ± S.E. (x 0.01mm, n = 10), and
* : Significant difference from normal group ($p < 0.05$),
** : ditto ($P < 0.01$), and
*** : ditto ($P < 0.001$).

Pharmacological Test Example 3

(Acute toxicity)

Acute toxicity of the polymers according to the invention was examined by using ICR mice and SD rats

to find that the value of $LD_{50}$ were over 1g/kg. Therefore, the polymers have very low toxicity and can be administered in safety.

Medicine Preparation Example 1 (Capsule)

Capsules were prepared in a conventional manner and in following prescription.

| Ingredients | Amount |
|---|---|
| Compound (Example 1) | 100 (mg) |
| Magnesium stearate | 5 |
| Lactose | Remainder |
| | 150 mg/capsule |

Medicine Preparation Example 2 (Tablet)

Tablets were prepared in a conventional manner and in following prescription.

| Ingredients | Amount |
|---|---|
| Compound (Example 2) | 100 (mg) |
| Sodium laulyl sulfate | 10 |
| Magnesium stearate | 5 |
| Polyvinyl pyrrolidone (K30) | 11 |
| Carboxymethylcellulose | 7 |
| Lactose | 60 |
| Corn starch | Remainder |
| | 210 mg/tablet |

Medicine Preparation Example 3 (Injection)

A solution for injection purpose was prepared in a conventional manner and in following prescription, and aseptically poured into a vial.

| Ingredients | Amount |
|---|---|
| Compound (Example 1) | 50 (mg) |
| Distilled water for injection | 50 |
| Benzyl alcohol | 5 |
| | 105 mg/vial |

## Claims

**1.** An organogermanium or organosilicon compound shown by the formula of

$[0_{1/2})_3Y\text{-}C(R_1)(R_2)\text{-}C(R_3)(R_4)\text{-}(CH_2)_m\text{-}P(=0)(0R_5)_2]_n$

wherein $R_1$, $R_2$, $R_3$, $R_4$ and $R_5$ are same or different and each represents hydrogen atom, an lower alkyl group with possible substituent(s) or phenyl radical with possible substituent(s), respectively; Y is germanium or silicon atom; $_m$ is an integer of 0 or more; and $_n$ is an integer of 2 or more,
or a salt thereof.

**2.** A process for the preparation of an organogermanium or organosilicon compound shown by the formula of

$[0_{1/2})_3Y\text{-}C(R_1)(R_2)\text{-}C(R_3)(R_4)\text{-}(CH_2)_m\text{-}P(=0)(0R_5)_2]_n$

wherein $R_1$, $R_2$, $R_3$, $R_4$ and $R_5$ are same or different and each represents hydrogen atom, an lower alkyl group with possible substituent(s) or phenyl radical with possible substituent(s), respectively; Y is germanium or silicon atom; $_m$ is an integer of 0 or more; and $_n$ is an integer of 2 or more,
or a salt thereof, which comprises steps of reacting a trihalogermane or trihalosilane shown by the formula of

$X_3YH$

wherein X and Y have the meanings as referred to, with a phosphonic acid derivative shown by the formula of

$(R_1)(R_2)C=C(R_3)\text{-}(CH_2)_m\text{-}P(=0)(0R_4)_2$

wherein $R_1$, $R_2$, $R_3$ and $R_4$ have the meanings as referred to,
subjecting to hydrolysis the resulting trihalogermylphosphonic acid derivative or trihalosilyphosphonic acid derivative shown by the formula of

$X_3Y\text{-}C(R_1)(R_2)\text{-}C(R_3)H\text{-}(CH_2)_m\text{-}P(=0)(0R_4)_2$

wherein X, Y, $R_1$, $R_2$, $R_3$ and $R_4$ have the meanings as referred to,
and if necessary, converting the derivative into its salt.

**3.** A process for the preparation of an organogermanium compound of the formula of

$[0_{1/2})_3Ge\text{-}C(R_6)(R_7)\text{-}C(R_8)(R_9)\text{-}(CH_2)_m\text{-}P(=0)(0R_{10})_2]_n$

wherein $R_6$, $R_7$, $R_8$, $R_9$ and $R_{10}$ are same or different and each represents hydrogen atom, an lower alkyl group with possible substituent(s), or phenyl radical with possible substituent(s), respectively; Y is germanium or silicon atom; $_m$ is an integer of 0 or more; and $_n$ is an integer of 2 or more,
or a salt thereof, which comprises steps of reacting dihalogermane shown by the formula of

$X_2Ge$

wherein X is halogen atom,
with a haloalkanephosphonic acid derivative shown by the formula of

$X\text{-}C(R_6)(R_7)\text{-}C(R_8)(R_9)\text{-}(CH_2)_m\text{-}P(=0)(0R_{10})_2$

wherein $R_6$, $R_7$, $R_8$, $R_9$, $R_{10}$ and $_m$ have the meanings as referred to,
subjecting the resulting trihalogermylalkanephosphonic acid derivative shown by the formula of

$X_3Ge\text{-}C(R_6)(R_7)\text{-}C(R_8)(R_9)\text{-}(CH_2)_m\text{-}P(=0)(0R_{10})_2$

wherein $R_6$, $R_7$, $R_8$, $R_9$, $R_{10}$ and $_m$ have the meanings as referred to,
and if necessary converting the derivative into its salt.

**4.** A pharmaceutical composition which comprises an effective amount of at least one substance selected from the group consisting of organogermanium and organosilicon compounds shown by the formula of

$[0_{1/2})_3Y\text{-}C(R_1)(R_2)\text{-}C(R_3)(R_4)\text{-}(CH_2)_m\text{-}P(=0)(0R_5)_2]_n$

wherein $R_1$, $R_2$, $R_3$, $R_4$ and $R_5$ are same or different and each represents hydrogen atom, an lower alkyl group with possible substituent(s) or phenyl radical with possible substituent(s), respectively; Y is germanium or silicon atom; $_m$ is an integer of 0 or more; and $_n$ is an integer of 2 or more,
and pharmacologically acceptable salts thereof, in addition to a pharmacologically acceptable carrier.

5. A pharmaceutical composition as claimed in Claim 4, which is administered to an individual requiring immune modulation.

European Patent Office

# EUROPEAN SEARCH REPORT

Application Number

EP 92 11 2362

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | GB-A-836 241 (GENERAL ELECTRIC COMPANY)<br>* the whole document *<br>--- | 1,2 | C07F9/38<br>A61K31/695<br>C08G77/30<br>C08G79/00<br>C07F9/40<br>A61K31/66<br>A61K31/80 |
| A | EP-A-0 404 062 (SANWA KAGAKU KENKYUSHO CO.,LTD.)<br>* page 2 - page 7 *<br>--- | 1,4,5 | |
| A | PATENT ABSTRACTS OF JAPAN<br>vol. 6, no. 202 (C-129)10 July 1982<br>& JP-A-57 111 320 ( TOKUYAMA SODA KK )<br>* abstract *<br>----- | 1,4,5 | |
| | | | TECHNICAL FIELDS SEARCHED (Int. Cl.5 ) |
| | | | C07F<br>C08G<br>A61K |

The present search report has been drawn up for all claims

| Place of search | Date of completion of the search | Examiner |
|---|---|---|
| THE HAGUE | 13 OCTOBER 1992 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)